# EUROPEAN PATENT APPLICATION

(11) **EP 4 533 996 A1**
(43) Date of publication of application: **09.04.2025**
(21) Application number: 24203933.7
(22) Date of filing: 01.10.2024
(51) Int. Cl.: A61B 1/002, G02B 23/24

(54) **MEDICAL INSTRUMENT**

(30) Priority: 04.10.2023 DE 102023126923
(71) Applicant: Karl Storz SE & Co. KG, 78532 Tuttlingen (DE)
(72) Inventor: Eisenlauer, Johannes, 78532 Tuttlingen (DE)

(57) **Abstract**

A medical instrument (10) comprises an elongated optical system (50) providing an optical axis (58) and comprising a number of rod lenses (52) relaying an image produced by an objective lens (24) near a distal end (12) of the medical instrument (10) to a proximal region (16) of the medical instrument (10), a carriage (60) mechanically coupled to a proximal end region (55) of the optical system (50) and a plurality of elastic arrangements (70) producing forces in distal directions. The carriage (60) transmits the forces to the proximal end region (55) of the optical system (50).

## Description

The present invention refers to a medical instrument comprising an optical system the proximal end of which needs to be biased in a distal direction. In particular, the present invention refers to a resectoscope or a similar medical instrument such as a shaver comprising a tube accommodating a working channel or the like and an optical system arranged close to the outer surface of the tube.

Many endoscopes and other medical instruments comprise an elongated optical system with several rod lenses relaying an image produced by an objective lens at the distal end of the medical instrument to a more proximal region, in particular to the proximal end of the shaft or to an ocular at a proximal end region of the instrument. Reusable medical instruments must be sterilized after use. Sterilization in an autoclave, providing a pure water vapor atmosphere at a temperature of approximately 140 degrees Centigrade, has many advantages. However, at the high temperature, the various components of the instrument expand differently. Therefore, many conventional endoscopes and other medical instruments with an elongated optical system comprise springs or other elastic elements holding the series of optical components together under a flexible compression. Frequently a coil spring is located between two rod lenses or at the proximal end of the elongated optical system in order to absorb expansion and contraction of the optical system during the autoclaving procedure, preventing, thereby, damage to the components of the optical system, in particular the delicate rod lenses. For example, EP 3381347 A1 shows an endoscope with an instrument shaft and an optical arrangement extending through the instrument shaft. The optical arrangement comprises optical components that are accommodated in a support shaft, which is arranged in the instrument shaft in an axially displaceable manner and is preloaded distally by two concentric coil springs.

An object of the present invention is to provide an improved medical instrument comprising an elongated optical system.

This object is solved by the subject matter of the independent claim.

Further embodiments are described in the dependent claims.

A medical instrument comprises an elongated optical system providing an optical axis and comprising a number of rod lenses relaying an image produced by an objective lens near a distal end of the medical instrument to a proximal region of the medical instrument; a carriage mechanically coupled to a proximal end region of the optical system; and a plurality of elastic arrangements producing forces in distal directions, wherein the carriage transmits the forces to a proximal end region of the optical system.

The image produced by the objective lens is a most distal real image. In particular, this most distal real image produced by the objective lens is relayed by a first relaying group of lenses producing a first real intermediate image from the most distal real image, a second relaying group of lenses producing a second real intermediate image from the first real intermediate image, and so on. Therein, the first intermediate real image is proximal to the most distal real image, the second intermediate real image is proximal to the first intermediate real image, and so on. The first relaying group of lenses is arranged between the most distal real image and the first intermediate real image, the second relaying group of lenses is arranged between the first intermediate real image and the second intermediate real image, and so on.

At the proximal end of the optical system, a most proximal intermediate real image is produced by a most proximal relaying group of lenses. This most proximal intermediate real image is usually viewed by a human through an ocular lens or eyepiece producing a virtual image from the most proximal intermediate real image. Alternatively, a camera can be coupled to the ocular lens or eyepiece, the camera capturing the virtual image produced by the ocular lens or eyepiece. As a further alternative, a lens or group of lenses with a positive total refractive power produces a last real image in the light sensitive layer of an image sensor from the most proximal intermediate real image.

The number of intermediate real images equals the number of relaying groups of lenses and can be any natural number. Every single relaying group of lenses inverts the image. In case of an odd number of relaying groups of lenses, the most proximal intermediate real image is inverted.

Throughout this specification, the optical system comprises all the relaying groups of lenses and produces said intermediate real images and said most proximal intermediate real image. In particular, the optical system does not comprise the ocular lens or eyepiece. As an alternative, the optical system can comprise the ocular lens or eyepiece or parts of the ocular lens or eyepiece, as well.

In particular, each relaying group of lenses consists of a pair of symmetrically arranged rod lenses. In particular, each rod lens is a doublet lens, glued or air-gapped.

In particular, the medical instrument comprises an elongated shaft extending from the distal end of the medical instrument to a proximal region. The distal end of the shaft can form the distal end of the medical instrument. The shaft may be rigid or partially or entirely flexible. The shaft may have a constant cross section throughout its length.

In particular, the proximal region is a proximal end region of the medical instrument. In particular, the medical instrument consists of the proximal region and a shaft extending from the proximal region to the distal end of the medical instrument. The proximal region may comprise or consist of a handle. For example, the handle comprises an ocular lens or eyepiece and/or another kind of interface to a camera, an image sensor or a camera comprising one or more image sensors, a light source providing illumination light, a connector receiving illumination light from a light source, a user interface receiving manual instructions at one or more dials, levers, switches, buttons, or other controls.

The medical instrument may comprise a bearing allowing for movements of a proximal end region of the optical system in a direction parallel to the optical axis of the optical system (in particular due to thermal expansion of the optical system) and guiding the proximal end region of the optical system in directions orthogonal to the optical axis of the optical system.

In particular, the bearing comprises or consists of an optics shaft holding the components of the optical system like a sleeve. In particular, the optics shaft is a tube essentially extending from the distal end of the optical system to the proximal end of the optical system. If the medical instrument comprises a shaft, the optics shaft can be arranged inside the shaft of the medical instrument. Cross sections of the components of the optical system are adapted to the clear cross section or inner cross section of the optics shaft, thereby providing a minimum clearance but low friction between the components of the optical system and the inner surface of the optics shaft. All components of the optical system - in particular rod lenses and tubes defining distances between rod lenses - can be merely slid into the optics shaft.

In particular, the optical system's proximal end region coupled to the carriage is formed by the most proximal component of the optical system, for example a rod lens. In particular, the carriage is coupled to the proximal end of the most proximal component of the optical system.

Coupling between the carriage and the proximal end region of the optical system can be provided by surface regions not being glued, soldered, welded, or otherwise permanently mechanically connected to each other in any other way but merely abutting on each other. In particular, the carriage can provide a distally oriented contact surface region merely abutting on a proximally oriented contact surface region of the proximal region of the optical system and exerting a distally directed force. The proximally oriented contact surface region can be a proximally oriented surface region of an optical component, for example a circular region of the proximal light exit surface of the most proximal rod lens. As an alternative, the proximally oriented contact surface region can be a proximally oriented surface region of a member mechanically connected to the proximal region of the optical system in a rigid and permanent manner. For example, the member is glued or welded to the lateral surface of the most proximal rod lens of the optical system.

As an alternative, the carriage can be rigidly and permanently coupled to the proximal region of the optical system. For example, the carriage is glued or soldered or welded to the proximal region of the optical system.

The plurality of elastic arrangements can comprise two, three or more elastic arrangements. All the elastic arrangements of the plurality of elastic arrangements can be equal and provide identical elastic characteristics. As an alternative, the plurality of elastic arrangements can comprise different elastic arrangements providing different elastic characteristics.

Each of the elastic arrangements can be a member or element or component separately produced and thereafter assembled with the carriage and other components of the medical instrument.

As an alternative, each of the elastic arrangements can be integrated with the carriage. Each of the elastic arrangements can be formed by an elastic region of the carriage. In particular, the elastic region of the carriage is shaped and operates like a conventional leaf spring.

Each of the plurality of elastic arrangements can be a spring made from elastic metallic material or from other material at least partially elastic. In particular, the elastic arrangements are coil springs. As an alternative, each of the plurality of elastic arrangements can be a pair of magnets, for example a pair of mutually repelling magnets.

In particular, each of the elastic arrangements is compressed (for example compression springs), and its length is reduced when the length of the optical system increases due to rising temperature. As an alternative, some or all of the plurality of elastic arrangements can be loaded with tension (for example tension springs or extension springs), their lengths being increased when the length of the optical system increases due to rising temperature. As another alternative, some or all of the plurality of elastic arrangements can be leaf springs or torsion bars or other types of elastic parts.

The forces produced by the elastic arrangements and the reaction force of the distal end region of the optical system impose a bending load on the carriage or on regions of the carriage. The carriage can be designed rigid to this bending load. As an alternative, elastic regions of the carriage can form the elastic arrangements or be part of the elastic arrangements.

The plurality of elastic arrangements and the carriage significantly reduce design restrictions, in particular restrictions to the design of the elastic arrangements. In particular, the diameters of coil springs can be large even when the proximal end region of the optical system is situated in a narrow space or very close to another member of the medical instrument. When, for example, the entire optical system is arranged parallel and next to a working channel, the diameter of a conventional coil spring pressing the proximal end surface of the optical system cannot be larger than the diameter of the optical system. When the forces of a plurality of elastic arrangements is transmitted to the proximal end region of the optical system by means of a carriage, the elastic arrangements can be arranged off axis and can be much larger. In this case, there are similar restrictions to the geometry of arrangements of the carriage. But the carriage, as a more or less rigid component, can be miniaturized more easily than a spring coil or other elastic member.

In a medical instrument as it is described herein, the plurality of elastic arrangements in particular provide similar mechanical characteristics, wherein each of the plurality of elastic arrangements is in mechanical contact with a respective one of a plurality of supporting points or supporting regions at a rigid part of the medical instrument, and wherein the plurality of supporting points or supporting regions are arranged symmetrical to the optical axis of the optical system.

In particular, all the supporting regions are similar. For example, in case of coil springs, the supporting regions can be circular or annular. Each of the supporting regions can be located in a respective recess geometrically defining the position of the respective end of the respective elastic arrangement. In case of a coil spring, a protrusion can be located inside the annular contact region, the protrusion geometrically defining the position of the end of the coil spring.

In a medical instrument as it is described herein, the plurality of elastic arrangements in particular are separately produced members, wherein the plurality of elastic arrangements provide similar mechanical characteristics, wherein the carriage is in mechanical contact with each of the plurality of elastic arrangements in a respective one of a plurality of contact regions, and wherein the plurality of contact regions are arranged symmetrical to the optical axis of the optical system.

In particular, the elastic arrangements provide identical mechanical characteristics. One end or one side of each of the elastic arrangements is in mechanical contact with the carriage, wherein the other end or other side of each of the elastic arrangements is in mechanical contact with a supporting point or supporting region at a rigid structure of the medical instrument.

In particular, all the contact points or contact regions are similar. For example, in case of coil springs, the contact regions can be circular or annular. Each of the contact points or contact regions can be located in a respective recess geometrically defining the position of the respective end of the respective elastic arrangement. In case of a coil spring, a protrusion can be located inside the annular contact region, the protrusion geometrically defining the position of the end of the coil spring.

In case of two elastic arrangements, the optical axis of the optical system is located between both supporting points or supporting regions, and the optical axis of the optical system and both supporting points or supporting regions are arranged in the same plane. In case of two elastic arrangements being separately produced members, the optical axis of the optical system is located between both contact points or contact regions, and the optical axis of the optical system and both contact points or contact regions are arranged in the same plane.

In case of three elastic arrangements, each of the supporting points defines one of three planes comprising the optical axis of the optical system. The angular distances between these three planes are 60 degrees.

In case of three elastic arrangements being separately produced members, each of the contact points defines one of three planes comprising the optical axis of the optical system. The angular distances between these three planes are 60 degrees.

As an alternative, the elastic arrangements, the supporting points or supporting regions and the contact points or contact regions, if applicable, are arranged asymmetrically.

In a medical instrument as it is described herein, the carriage is in particular a yoke extending in a direction substantially orthogonal to the optical axis of the optical system, wherein a central region of the carriage is mechanically coupled to the proximal end of the optical system, and wherein each of the ends of the carriage is mechanically biased in a distal direction by at least one of the plurality of elastic arrangements.

In particular, the yoke is a rigid beam, wherein the cross section of the beam can be rectangular or provide any other shape, and wherein the cross section is essentially constant along the beam or varies continuously or abruptly. Deviations from a simple beam shape may be required by the mechanical coupling of the central region of the carriage to the proximal end region of the optical system. Further deviations from a simple beam shape may be required by the contact regions mechanically coupled or even connected to respective ends of the elastic arrangements.

A medical instrument as it is described herein, in particular further comprises a tube at least one of forming a working channel for insertion of other medical instruments, a fluid channel for supplying a fluid to the distal end of the medical instrument, and a fluid channel facilitating suction of fluids from the distal end of the medical instrument, wherein the optical system is arranged adjacent to the outer surface of the tube.

In particular, the tube is cylindrical with a circular, elliptical or other cross section, and the longitudinal axis or axis of symmetry of the tube is parallel to the optical axis of the optical system.

In particular, the optical system is arranged next to the outer surface of the tube, rod lenses or an optics shaft housing the rod lenses abutting on the outer surface of the tube. In this situation, a conventional design with a coil spring directly biasing the proximal end of the optical system in the distal direction would require a large diameter of the optical system. However, in many applications the diameter of the optical system is reduced as much as possible in order to maximize the ratio between the inner cross section of the tube and the outer cross section of the shaft of the medical instrument. When the diameter of the optical system is less than 2 mm, a mechanically robust coil spring with a corresponding diameter and reproducible mechanical characteristics is a challenge. The carriage transmitting the forces produced by the elastic arrangements allows for elastic arrangements with dimensions largely independent from the diameter of the optical system even when the optical system is arranged next to a tube.

In a medical instrument as it is described herein, the carriage in particular comprises a surface region with a shape corresponding to the shape of the outer surface of the tube.

In this case, the tube can serve as a rail guiding the carriage.

For example, when the tube (more precisely: the outer surface of the tube) is a circular cylinder with a predetermined first diameter, the surface region is a cutout of a circular cylinder surface with a second diameter equal to or slightly larger than the first diameter.

A medical instrument as it is described herein in particular further comprises a reflective interface reflecting the relayed image away from the optical axis of the optical system,
wherein the reflective interface is located proximal to the carriage.

The reflective interface can be a first-surface mirror, a second-surface mirror, an interface reflecting based on total internal reflection or any other reflective interface.

In particular, the normal to the reflective interface and the optical axis of the optical system enclose angles of 45 degrees and 135 degrees, wherein the optical axis is angled 90 degrees by the reflecting interface. If the medical instrument provides a shaft, the optical axis is in particular bent away from the center of the shaft. If the medical instrument provides a tube forming a working channel or a fluid channel, the optical axis is in particular bent away from the tube.

In particular, the position of the reflective interface located downstream the proximal end of the optical system is adjusted during manufacture or maintenance, but stationary when used, cleaned and autoclaved by a customer. In this case, the position of the optical axis downstream the reflective interface is not altered by any axial movement of the proximal end region of the optical system.

In a medical instrument as it is described herein, the carriage in particular comprises a central through hole or recess accommodating the proximal end of the optical system.

In particular, the cross section of the through hole or recess is adapted to the cross section of the proximal end of the optical system.

The through hole can provide a collar. Proximal to the collar, the cross section of the through hole is slightly reduced and smaller than the cross section of the proximal end of the optical system. In this case, the collar can form the above-mentioned contact region mechanically coupling the carriage to the proximal end of the optical system. Analogously, a base of a recess can form the above-mentioned contact region mechanically coupling the carriage to the proximal end of the optical system.

As an alternative, the inner surface of the through hole can be glued or soldered or welded to the proximal end region of the optical system.

In a medical instrument as it is described herein, the carriage is in particular rigidly connected to the proximal end of the optical system.

For example, the rigid connection can be formed by tight fit, adhesive, solder, weld, etc.

When the carriage is rigidly connected to the proximal end of the optical system, there is no need for both the proximal end region of the optical system and the carriage to be guided. When the proximal end region of the optical system is guided - for example in a tube or shaft with corresponding cross section - no linear motion bearing or other means guiding the carriage is required. When the carriage is guided by any kind of linear motion bearing or other means guiding the carriage, no bearing guiding the proximal end region of the optical system is required.

A medical instrument as it is described herein, in particular further comprises a kinematic pair mechanically coupling the proximal end region of the optical system to the carriage, the kinematic pair allowing for at least one of translational movement of the carriage relative to the proximal end of the optical system in at least one direction orthogonal to the optical axis of the optical system and rotation of the carriage about at least one axis orthogonal to the optical axis of the optical system relative to the proximal end of the optical system.

In particular, the kinematic pair forms a hinge or joint reducing or preventing the transmittal of forces orthogonal to the optical axis of the optical system and of torque.

In particular, the kinematic pair is formed by a first surface area of the carriage and a second surface area at the proximal end region of the optical system or at a member rigidly connected to the proximal end region of the optical system. The first surface area abuts on the second surface area in at least one point, one line or in a two-dimensional area. In particular, the mechanical contact allows for transfer of forces orthogonal to the first and second surface areas. In particular, rotation is allowed in a small angular region of several degrees or more.

In particular, the translational and/or rotational movement of the carriage relative to the proximal end region of the optical system considerably reduces transfer of unwanted or undesirable forces orthogonal to the optical axis. This can reduce the risk of canting of the proximal end region of the optical system, for example canting of the most proximal rod lens in the optics shaft.

A medical instrument as it is described herein, in particular further comprises a bearing allowing for movements of the carriage in a direction parallel to the optical axis of the optical system and guiding the carriage in directions orthogonal to the optical axis of the optical system.

Such bearing can reduce the transfer of dynamically generated lateral forces to the proximal end region of the optical system.

In a medical instrument as it is described herein, the plurality of elastic arrangements are in particular coil springs, wherein the carriage comprises at least one of a protrusion accommodated in one end of one of the plurality of coils springs and a recess accommodating one end of one of the plurality of coil springs.

Form fit or positive locking between one end of a coils spring and the carriage facilitates fast and easy assemblage and considerably reduces unwanted displacement of the coil spring.

In a medical instrument as it is described herein, the axes of the coil springs are in particular parallel to the optical axis of the optical system.

In particular, a medical instrument as it is described herein, is a shaver, a resectoscope, or comprises a working channel for receiving a shaver or a resectoscope. A shaver is a medical instrument for surgically removing tissue with a rotating blade. A resectoscope is a medical instrument for surgically removing tissue using an electrical current.

### Short description of the figures

Embodiments will be described below with reference to the enclosed figures.
- Figure 1: shows a schematic representation of a medical instrument;
- Figure 2: shows a schematic representation of a longitudinal section through a proximal region of the medical instrument shown in figure 1;
- Figure 3: shows a schematic representation of another longitudinal section through the proximal region of the medical instrument shown in figures 1 and 2;
- Figure 4: shows a schematic representation of a transversal section through the proximal region of the medical instrument shown in figures 1 through 3;
- Figure 5: shows a schematic representation of a longitudinal section through the proximal region of another medical instrument;
- Figure 6: shows a schematic representation of a longitudinal section through the proximal region of another medical instrument.

### Description of embodiments

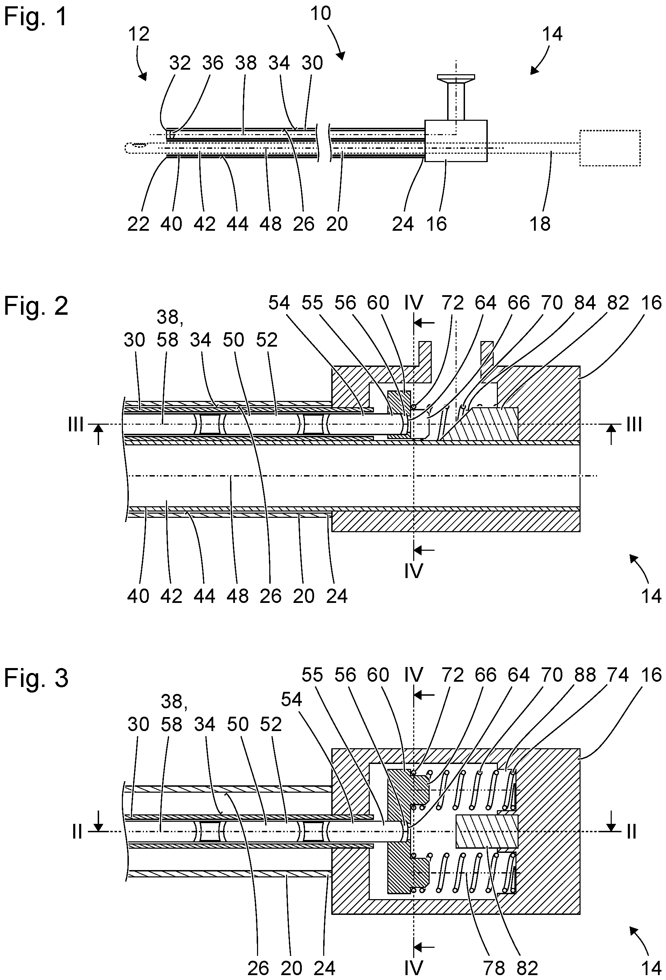

Figure 1 shows a schematic presentation of a medical instrument 10 with a distal end 12 and a proximal end 14 formed by a handle 16 as a proximal region of the medical instrument 10. In the example shown in figure 1, an eyepiece is provided at the handle 16. The medical instrument 10 either comprises or can accommodate a shaver 18. In figure 1, the shaver 18 is shown in dashed lines, because the shaver 18 can be both a permanent part of the medical instrument or can be inserted into the medical instrument during its application in a medical procedure only.

The medical instrument 10 comprises a shaft 20. The shaft 20 and components located within the shaft 20 are shown in a longitudinal section, whereas the rest of the medical instrument and the Bigatti shaver are shown in a side view.

In the example shown, the shaft 20 is formed by a straight and rigid tube providing a circular cross section. A proximal end 24 of the shaft 20 can be permanently mechanically connected to the handle 16 of the medical instrument 10. As an alternative, a releasable coupling not shown in Figure 1 can be provided between the proximal end 24 of the shaft 20 and the handle 16.

A distal end 22 of the shaft 20 forms the distal end 12 of the medical instrument 10 or is arranged close to the distal end 12 of the medical instrument 10. When the shaver 18 is not part of the medical instrument 10, the distal end 22 of the shaft 20 forms the distal end 12 of the medical instrument. If the shaver 18 is a permanent component of the medical instrument 10, the distal end of the shaver 18 forms the distal end 12 of the medical instrument 10.

An optics shaft 30 formed by a tube with circular cross section is arranged in the shaft 20. The cross section of the optics shaft 30 is much smaller than the cross section of the shaft 20. In the example shown, a distal end 32 of the optics shaft 30 is essentially flush with the distal end 22 of the shaft 20. As an alternative, the distal end 32 of the optics shaft 30 can protrude from the distal end 22 of the shaft 20. As a further alternative, the distal end 32 of the optics shaft 30 can be retracted relative to the distal end 22 of the shaft 20.

An outer surface 34 of the optics shaft 30 abuts on an inner surface 26 of the shaft 20 in a straight line. The outer surface 34 of the optics shaft 30 can be permanently connected to the inner surface 26 of the shaft 20, for example by a welded, soldered, or adhesive joint.

An objective lens 36 generating a real image of objects located distal to the distal end 32 of the optics shaft 30 is located at and hermetically closes the distal end 32 of the optics shaft 30. In particular, the optical axis of the objective lens 36 is identical to the axis of symmetry 38 of the optics shaft 30. An optical system is arranged in the optics shaft 30 but not shown in figure 1.

A working channel tube 40 is arranged in the shaft 20. In the present example, the cross section of the working channel tube 40 is circular. The lumen 42 of the working channel tube 40 forms a working channel accommodating a shaft of the shaver 18. As an alternative, the lumen 42 of the tube 40 can be part of a fluid conduit for supplying fluid to the distal end 12 of the medical instrument 10 or for facilitating suction of fluids from the distal end 12 of the medical instrument 10. As an alternative, the lumen 42 of the tube 40 can serve as a conduit for other tools involved in a surgical intervention.

An outer surface 44 of the working channel tube 40 abuts on the outer surface 34 of the optics shaft 30 in a straight line. The outer surface 44 of the working channel tube 40 can be permanently mechanically connected to the outer surface 34 of the optics shaft 30, for example by a welded, soldered, or adhesive joint. As an alternative, the working channel shaft 40 can be separated from the medical instrument 10, for example withdrawn in a proximal direction. The sum of the outer diameter of the optics shaft 30 and the outer diameter of the working channel tube 40 equals or is slightly smaller than the inner diameter of the shaft 20. The axis of symmetry 38 of the optics shaft 30 and the axis of symmetry 48 of the working channel tube 40 are parallel.

Figure 2 shows a schematic and enlarged representation of a longitudinal section through the handle 16 of the medical instrument 10. The sectional plane is parallel to the plane of projection of figure 1 and comprises the axis of symmetry 38 of the optics shaft 30 and the axis of symmetry 48 of the working channel tube 40.

In figure 2, it is visible that the optics shaft 30 abuts on the working channel tube 40.

The optics shaft 30 contains and holds an optical system 50 comprising a plurality of rod lenses 52. The position of a distal end of the optical system 50 is defined by the position of the objective lens 36 rigidly mechanically connected to the distal end 32 of the optics shaft 30 (cf. figure 1 and respective description). Thin-walled tube-like spacers (no reference numeral in figure 1) with predefined lengths are positioned between neighboring rod lenses 52. A proximal end region 55 of the optical system 50 is formed by the most proximal rod lens 54 of the optical system 50. The optical axis 58 of the optical system 50 is identical to the axis of symmetry 38 of the optics shaft 30.

The material of the optics shaft 30 and the materials of the rod lenses 52, 54 provide different coefficients of thermal expansion. Therefore, heating of the medical instrument 10 during an autoclave procedure causes different thermal expansion of the optics shaft 30 and the optical system 50 and movement of the proximal end region 55 and the proximal end 56 of the optical system 50 relative to the proximal end of the optics shaft 30.

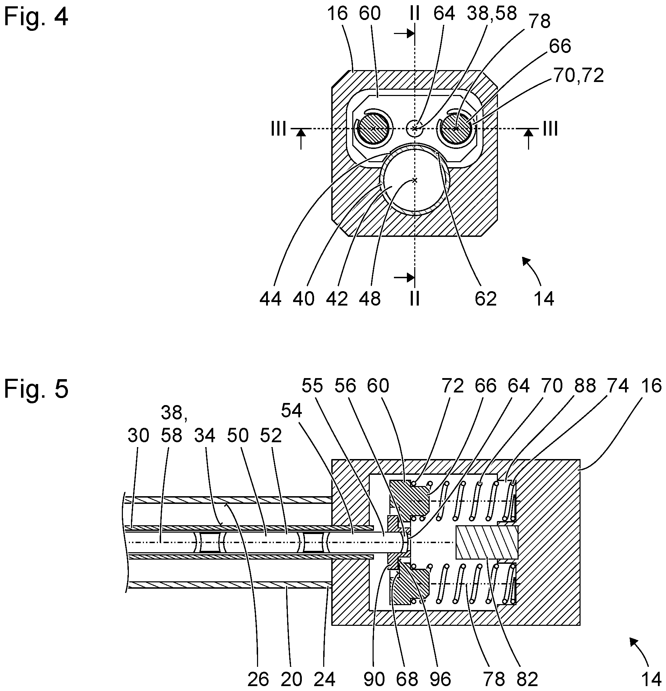

The proximal end region 55 of the optical system 50 is guided in the optics shaft 30 and mechanically coupled to a carriage 60. In the present example, the proximal end region 55 of the optical system is accommodated in a central through hole 64 in the carriage 60. As described in more detail below with reference to Figures 3 and 4, the carriage 60 provides the shape of a yoke extending in a direction perpendicular to the sectional plane of figure 2.

The carriage 60 is symmetrical to the sectional plane of figure 2. The carriage 60 comprises two protrusions 66 formed like pins or studs protruding in a proximal direction. One of these protrusions 66 can be seen in figure 2. Each of the protrusions 66 is accommodated in a distal end 72 of a respective spring coil 70, which serves as elastic arrangement. Accordingly, each of the protrusions 66 defines a contact region of the carriage 60 that is in mechanical contact with the respective elastic arrangement. In figure 3, the proximal end of the coil spring 70 is covered.

A prism 82 provides a reflective interface 84. In the example shown, the reflective interface 84 is a first surface mirror, and the angle between the optical axis 58 of the optical system 50 and the reflective interface 84 is 45 degrees. Therefore, the reflective interface 84 deflects the optical axis 58 of the optical system 50 by 90 degrees.

Figure 3 shows a schematic representation of another longitudinal section through the handle 16 of the medical instrument 10. The sectional plane III-III of figure 3 is orthogonal to the sectional plane II-II of figure 2 and comprises the axis of symmetry 38 of the optics shaft 30 and the optical axis 58 of the optical system 50 accommodated in the optics shaft 30. The position of the sectional plane III-III of figure 3 is indicated in figure 2. The position of the sectional plane II-II of figure 2 is indicated in figure 3.

The carriage 60 is mirror image symmetrical to the sectional plane II-II of figure 2. The cross section of the central through hole 64 of the carriage 60 is reduced at the proximal end of the central through hole. The resulting collar supports the rim of the proximal end 56 of the optical system 50.

The axes of symmetry 78 of both coil springs 70 are parallel to the optical axis 58 of the optical system 50. The axes of symmetry 78 of both coil springs 70 and the optical axis 58 of the optical system 50 are in the same plane, namely in the sectional plane III-III of figure 3. The distances of the axes of symmetry 78 of both coil springs 70 from the optical axis 58 of the optical system 50 are equal. Both coil springs 70 provide the same geometrical and mechanical characteristics.

The proximal end 74 of each of the coil springs 70 is accommodated in a respective recess 88 in a support rigidly connected to the handle 16, in particular in a body member of the handle 16 providing an interior space for accommodating at least the proximal end 56 of the optical system, the carriage 60 and the elastic arrangements in form of the coils springs 70. Accordingly, the recesses 88 each provide a supporting region at a rigid part of the medical instrument, i. e. at body member of the handle 16. Each of the coil springs 70 is compressed between the respective recess 88 and the carriage 60. Therefore, both coil springs bias the carriage 60 in a distal direction. The carriage 60 transfers this bias force to the proximal end 56 of the optical system 50.

Figure 4 shows a schematic representation of a transverse section through the handle 16 of the medical instrument 10. The sectional plane IV-IV of figure 4 is orthogonal to the sectional plane II-II of figure 2, orthogonal to the sectional plane III-III of figure 3 and orthogonal to the axis of symmetry 38 of the optics shaft 30 and the optical axis 58 of the optical system 50 accommodated in the optics shaft 30. The position of the sectional plane IV-IV of figure 4 is indicated in figures 2 and 3. The position of the sectional plane II-II of figure 2 and the position of sectional plane III-III of figure 3 are indicated in figure 4.

The carriage 60 provides the shape of a yoke extending orthogonal to the axis of symmetry 38 of the optics shaft 30 which is identical to the optical axis 58 of the optical system 50. The axis of symmetry 38 of the optics shaft 30 and the optical axis 58 of the optical system 50 and the axes of symmetry 78 of the coil springs 70 are in the same plane, namely the sectional plane III-III of figure 3.

The carriage 60 provides a conformal surface region 62 conformal to the outer surface 44 of the working channel tube 40. In the example shown, there is a thin air gap between the conformal surface region 62 of the carriage 60 and the outer surface 44 of the working channel tube 40. This small gap avoids friction when the carriage 60 moves.

As an alternative, the conformal region 62 abuts on and slides against the outer surface 44 of the working channel tube 40. In this case, the conformal surface region 62 and the outer surface 44 of the working channel tube 40 form a linear-motion bearing guiding the carriage 60 along a predetermined path parallel to the axis of symmetry 48 of the working channel tube.

Figure 5 shows a schematic representation of a longitudinal section through the handle 16 of another medical instrument 10. The sectional plane of figure 5 is similar to the sectional plane III-III of figure 3 and comprises the axis of symmetry 38 of the optics shaft 30 and the optical axis 58 of the optical system 50 accommodated in the optics shaft 30 and the axes of symmetry 78 of the spring coils 70.

Many features, characteristics, and functions of the embodiment shown in figure 5 are similar to features, characteristics and functions of the embodiment described with reference to figures 1 through 4.

The embodiment shown in figure 5 differs from the embodiment described with reference to figures 1 through 4 in that the carriage 60 is neither rigidly connected nor directly coupled to the proximal end region 54 of the optical system 50. Rather, a cap or sleeve 90 is rigidly connected to the proximal end region 54 of the optical system 50. The sleeve 90 and the proximal end region 55 of the optical system 50 can be mechanically connected by adhesive, solder or a weld or in another manner. As an alternative, the proximal end region 54 of the optical system 50 is merely accommodated in the sleeve 90. In this case, an inwardly directed collar at the proximal end of the sleeve 90 supports a circular rim at the proximal end 56 of the optical system 50.

A distally oriented surface region 68 of the carriage 60 abuts on a proximally oriented surface region 96 of the sleeve 90. Thereby, the bias produced by the spring coils 70 is transferred from the carriage 60 to the sleeve 90 and the proximal end region 54 of the optical system 50.

The distally oriented surface region 68 of the carriage 60 can slide on the proximally oriented surface region 96 of the sleeve 90 in lateral directions. Therefore, forces orthogonal to the optical axis 58 of the optical system 50 cannot be transferred from the carriage 60 to the sleeve 90 and the proximal end region 55 of the optical system 50. Only force parallel to the optical axis 58 of the optical system 50 can be transferred to the proximal end region 55 of the optical system 50. This protects the optical system 50 from detrimental loads.

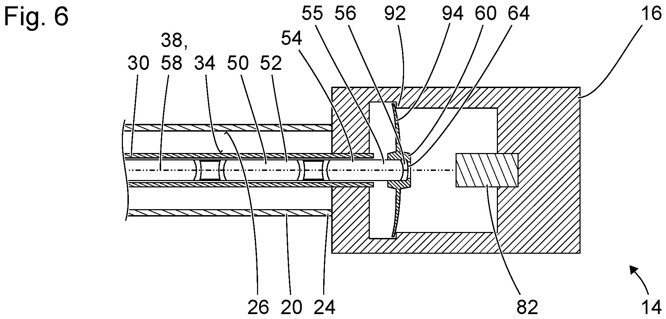

Figure 6 shows a schematic representation of a longitudinal section through the handle 16 of another medical instrument 10. The sectional plane of figure 6 is similar to the sectional planes of figures 3 and 5 and comprises the axis of symmetry 38 of the optics shaft 30 and the optical axis 58 of the optical system 50 accommodated in the optics shaft 30.

Many features, characteristics, and functions of the embodiment shown in figure 6 are similar to features, characteristics and functions of the embodiments described with reference to figures 1 through 5.

In embodiments described above with reference to figure 1 through 5, two coil springs 70 bias the carriage 60 in a distal direction. In the embodiment shown in figure 6, steps 92 are provided in the handle 16. In particular, the steps 92 are provided at side walls of the interior space of the handle, that are parallel to the optical axis 58. The elastic arrangements are formed by elastic bars 94, The ends of the elastic bars 94 are supported by the steps 92. Each step 92 serves as a supporting point for a respective one of the elastic bars 94. In the example shown, the elastic bars 94 are integral parts of the carriage 60 and define elastic regions of the carriage 60. The elastic bars 94 are elastic due to their geometry, in particular due to thin cross sections. The elastic bars 94 form a kind of leaf springs.

As an alternative, other elastic arrangements can be used, for example members made from elastomer or leaf springs. More than two elastic arrangements can be used, for example three or four elastic arrangements. The elastic arrangements can provide different geometrical and mechanical features. However, a plurality of elastic arrangements not exerting a net moment on the proximal end region 54 of the optical system 50 is advantageous.

### List of reference numerals

- **10**: **medical instrument**
- 12: distal end of the medical instrument 10
- 14: proximal end of the medical instrument 10
- 16: handle near the proximal end 14 of the medical instrument 10
- 18: Bigatti shaver

- **20**: **shaft** of the medical instrument 10
- 22: distal end of the shaft 20
- 24: proximal end of the shaft 20
- 26: inner surface of the shaft 20

- **30**: **optics shaft** in the shaft 20
- 32: distal end of the optics shaft 30
- 34: outer surface of the optics shaft 30
- 36: objective lens at the distal end 32 of the optics shaft 30
- 38: axis of symmetry of the optics shaft 30

- **40**: **working channel tube** in the shaft 20
- 42: lumen of the working channel tube 40 forming a working channel or a fluid duct
- 44: outer surface of the working channel tube 40
- 48: axis of symmetry of the lumen 42 of the working channel tube 40

- **50**: **optical system** of the medical instrument 10
- 52: rod lens of the optical system 50
- 54: most proximal rod lens
- 55: proximal end region of the optical system 50
- 56: proximal end of the optical system 50
- 58: optical axis of the optical system 50

- **60**: **carriage**
- 62: conformal surface region of the carriage 60
- 64: central through hole of the carriage 60
- 66: protrusion of the carriage 60
- 68: distally oriented surface region of the carriage 60, abutting on proximally oriented surface region 96 of the sleeve 90

- **70**: **spring coil**
- 72: distal end of the spring coil 70
- 74: proximal end of the spring coil 70
- 78: axis of symmetry of the spring coil 70

- 82: prism
- 84: reflective interface at the prism 82
- 88: recess in the support, accommodating the proximal end 72 of the spring 70

- **90**: **sleeve** at the proximal end 56 of optical system 50
- 92: supporting step in the handle 16
- 94: elastic bars of the carriage 60
- 96: proximally oriented surface region of the sleeve, abutting on distally oriented surface region 68 of the carriage 60

## Claims

1. Medical instrument (10) comprising:
an elongated optical system (50) providing an optical axis (58) and comprising a number of rod lenses (52) relaying an image produced by an objective lens (24) near a distal end (12) of the medical instrument (10) to a proximal region (16) of the medical instrument (10);
a carriage (60) mechanically coupled to a proximal end region (55) of the optical system (50),
a plurality of elastic arrangements (70) producing forces in distal directions,
wherein the carriage (60) transmits the forces to the proximal end region (55) of the optical system (50).

2. Medical instrument (10) according to claim 1, wherein the elastic arrangements (70) are arranged off axis of the optical axis (58) of the optical system (50).

3. Medical instrument (10) according to claim 1 or 2, wherein
each of the plurality of elastic arrangements (70) is in mechanical contact with one of a plurality of supporting points (92) or supporting regions (88) at a rigid part of the medical instrument, and
the plurality of supporting points (92) or supporting regions (88) are arranged symmetrical to the optical axis (58) of the optical system (50).

4. Medical instrument (10) according to any of the preceding claims, wherein
the plurality of elastic arrangements (70) are separately produced members,
the carriage (60) is in mechanical contact with each of the plurality of elastic arrangements (70) in one of a plurality of contact regions, and
the plurality of contact regions are arranged symmetrical to the optical axis (58) of the optical system (50).

5. Medical instrument (10) according to any of the preceding claims, wherein the plurality of elastic arrangements (70) are equal.

6. Medical instrument (10) according to the preceding claim, wherein
the carriage (60) is a yoke extending in a direction substantially orthogonal to the optical axis (58) of the optical system (50),
a central region (64) of the carriage (60) is mechanically coupled to the proximal end region (55) of the optical system (50),
each of the ends of the carriage (60) is mechanically biased in a distal direction by at least one of the plurality of elastic arrangements (70).

7. Medical instrument (10) according to any of the preceding claims, further comprising:
a tube (40) at least one of forming a working channel for insertion of another member of the medical instruments or other medical instruments, a fluid channel for supplying a fluid to the distal end (12) of the medical instrument (10), and a fluid channel facilitating suction of fluids from the distal end (12) of the medical instrument (10),
wherein the optical system (50) is arranged adjacent to an outer surface (44) of the tube (40).

8. Medical instrument (10) according to the preceding claim, wherein
the carriage (60) comprises a surface region (62) with a shape corresponding to the shape of the outer surface (44) of the tube (40).

9. Medical instrument (10) according to any of the preceding claims, further comprising:
a reflective interface (84) reflecting the relayed image away from the optical axis (58) of the optical system (50),
wherein the reflective interface (84) is located proximal to the carriage (60).

10. Medical instrument (10) according to any of the preceding claims, wherein
the carriage (60) comprises a central through hole (64) or recess accommodating the proximal end (55) of the optical system (50).

11. Medical instrument (10) according to any of the preceding claims, wherein
the carriage (60) is rigidly connected to the proximal end (56) of the optical system (50).

12. Medical instrument (10) according to any of the preceding claims, wherein
the plurality of elastic arrangements (70) are coil springs,
the carriage (60) comprises at least one of a protrusion (66) accommodated in one end (72) of one of the plurality of coils springs (70) and a recess accommodating one end of one of the plurality of coil springs (70).

13. Medical instrument (10) according to the preceding claim, wherein axes (78) of the coil springs (70) are parallel to the optical axis (58) of the optical system (50).

14. Medical instrument (10) according to any of the preceding claims, wherein elastic regions (94) of the carriage can form the elastic arrangements (70) or be part of the elastic arrangements (70).

15. Medical instrument (10) according to any of the preceding claims, wherein the plurality of elastic arrangements (70) are leaf springs.

16. Medical instrument (10) according to any of the preceding claims, further comprising
a kinematic pair mechanically coupling the proximal end region (55) of the optical system (50) to the carriage (60) for translational movement of the carriage (60) relative to the proximal end (55) of the optical system (50) in at least one direction orthogonal to the optical axis (58) of the optical system (50) and/or for rotation of the carriage (60) about at least one axis orthogonal to the optical axis (58) of the optical system (50) relative to the proximal end (55) of the optical system (50).

17. Medical instrument (10) according to any of the preceding claims, wherein
a kinematic pair is formed by a first surface area (68) of the carriage (60) and a second surface area (96) at the proximal end region (55) of the optical system (50) or at a member rigidly connected to the proximal end region (55) of the optical system (50), and
the first surface area (68) abuts on the second surface area (96) for transfer of forces orthogonal to the first surface area (68) and second surface area (96) and/or rotation of the first surface area (68) relative to the second surface area (96).

18. Medical instrument (10) according to any of the preceding claims, wherein
the medical instrument (10) is a shaver or a resectoscope or comprises a working channel (42) for receiving a shaver or a resectoscope.
